## Europäisches Patentamt
## European Patent Office
## Office européen des brevets

(19)

(11) Veröffentlichungsnummer: **0 090 205**
**B1**

(12) ## EUROPÄISCHE PATENTSCHRIFT

(45) Veröffentlichungstag der Patentschrift:
11.09.85

(51) Int. Cl.⁴: **A 01 N 59/12**

(21) Anmeldenummer: 83102216.5

(22) Anmeldetag: 07.03.83

(54) Präparat zur Euterpflege und Zitzendesinfektion bei Milchvieh.

(30) Priorität: 15.03.82 DE 3209328

(43) Veröffentlichungstag der Anmeldung:
05.10.83 Patentblatt 83/40

(45) Bekanntmachung des Hinweises auf die Patenterteilung:
11.09.85 Patentblatt 85/37

(84) Benannte Vertragsstaaten:
AT BE CH DE FR GB IT LI NL SE

(56) Entgegenhaltungen:
EP - A - 0 017 639
FR - A - 2 105 151
US - A - 3 663 694
US - A - 3 728 449

Janistyn, Handbuch der Kosmetika und Riechstoffe
"Paraffinöle"

(73) Patentinhaber: Henkel Kommanditgesellschaft auf Aktien, Postfach 1100 Henkelstrasse 67,
D-4000 Düsseldorf-Holthausen (DE)

(72) Erfinder: Lauermann, Georg, Dr., Burscheiderweg 123,
D-4021 Mettmann (DE)
Erfinder: Scholz, Siegfried, Dr., Benrodestrasse 23,
D-4000 Düsseldorf 13 (DE)
Erfinder: Koch, Ferdinand, Händelstrasse 36,
D-4010 Hilden (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**0 090 205**

## Beschreibung

Infektiöse Euterentzündungen (Rindermastitiden), wie sie besonders bei Hochleistungsrindern auftreten, sind mit erheblichen Milchverlusten verbunden und führen oft zu vorzeitiger Schlachtung der Tiere, sofern die Mastitis einen chronischen Verlauf mit daraus entstehender Drei- oder gar Zweistrichigkeit (Ausfall einzelner Euterviertel) nimmt.

Zur Infektionsvorbeuge wird das Eintauchen der Zitzen nach dem Melken in eine wirksame Desinfektionslösung empfohlen, wodurch die an der Zitzenhaut anhaftenden Schmutz- und Krankheitskeime besonders im Bereich der Zitzenöffnungen vermindert werden. Die Applikation erfolgt in Form niedrigviskoser wäßriger Lösungen, die zur Sicherstellung einer ausreichenden Wirksamkeit einen relativ hohen Gehalt an Desinfektionswirkstoffen enthalten. Da diese mehr oder weniger hautreizend sind, enthalten die Mittel in der Regel hautpflegende Substanzen. Solche Mittel sind beispielsweise beschrieben in US-PS 3 993 777 und Deutsche Tierärztliche Wochenschrift 86 (1979), Seite 85—88.

Die genannten Zitzentauchmittel eignen sich jedoch nicht zur Euterpflege, da die Euterhaut im Gegensatz zur Zitzenhaut gegen Desinfektionsmittel äußerst empfindlich ist, so daß es leicht zu Hautreizungen kommt. Daher werden zur Euterpflege spezielle Melkfette und -emulsionen angeboten, die die Euterhaut geschmeidig halten und Feuchtigkeitsverluste verhindern. Auch rauhe und rissige Euterhaut kann damit zur Schmerzlinderung behandelt werden. Zur Mastitisabwehr sind diese Mittel jedoch mangels einer ausreichenden Desinfektionswirkung ungeeignet.

Die Erfindung hat zum Ziel, ein Mittel zu schaffen, daß sich sowohl als Eintauchmittel zur Zitzendesinfektion nach dem Melken wie auch zur Pflege der empfindlichen Euterhaut eignet und damit die Anwendung zweier getrennter Mittel überflüssig macht. Dies wird erreicht durch die Bereitstellung eines flüssigen Mittels mit erhöhter Viskosität und einem darauf abgestimmten Gehalt an Desinfektionsmittel, wodurch beim Tauchvorgang eine ausreichende Desinfektionsmittelmenge an die Zitze gebracht, Reizungen der Euterhaut aber vermieden werden.

Gegenstand der Erfindung ist somit ein Präparat zur Zitzendesinfektion und Euterpflege bei Milchvieh, insbesondere Rindern, auf der Basis von jodhaltigen Desinfektionsmitteln und hautpflegenden Substanzen, dadurch gekennzeichnet, daß das Präparat eine wäßrige Emulsion mit erhöhter Viskosität folgender Zusammensetzung ist:

| | |
|---|---|
| 84,4—91,6 Gew.-% | salzfreies Wasser |
| 3—5 Gew.-% | Glycerin |
| 3—5 Gew.-% | Paraffinöl |
| 1—2,5 Gew.-% | gesättigte $C_{16}$—$C_{18}$-Fettalkohole |
| 1—2,5 Gew.-% | nichtionogener Emulgator |
| 0,4—0,6 Gew.-% | desinfizierend wirkender Jodkomplex |

wobei der Gehalt an aktivem Jod 800—1200 ppm und die Viskosität 25—35 sec/20°C im Fordbecher/ 4 mm-Düse beträgt (entspricht etwa 45—90 m Pa · s).

Das Präparat ist eine wäßrige Emulsion mit in engen Grenzen vorgegebener Viskosität und einem darauf abgestimmten Gehalt an einem desinfizierend wirkenden Jod-Komplex. Die erhöhte Viskosität bewirkt, daß beim Tauchen der Zitzen eine für die Desinfektionswirkung ausreichende Menge an Flüssigkeit an der Zitze hängen bleibt und der Strichkanal sicher verschlossen wird. Dem Enstehen einer Mastitis wird dadurch mit Sicherheit vorgebeugt. Infolge des niedrigen Gehaltes an Desinfektionsmittel werden Hautreizungen bei Applikation am Euter vermieden. Die hautpflegende Wirkung des Präparates kommt sowohl an der Zitze wie auch am Euter zur Geltung. Rückstandsprobleme, wie sie bei Anwendung höher konzentrierter Mittel auftreten können, werden weitgehend vermieden.

Der pH-Wert des Präparates ist demjenigen der gesunden Haut angepaßt (pH-Wert 2,5—3,5, vorzugsweise 3). Die Viskosität entspricht einer Durchlaufzeit von 25—35 Sekunden/20°C im Fordbecher mit 4 mm Düsenöffnung (entspricht etwa 45—90 m Pa · s).

Die Bestandteile des Präparates haben folgende Funktion:

Salzfreies Wasser (Kondenswasser) ist Lösungs- und Verdünnungsmittel.
Glycerin (Arzneibuchqualität, DAB 7) dient als Feuchthaltemittel für die Haut.
Paraffinöl (Arzneibuchqulität, DAB 8) hält die Haut geschmeidig.

Die gesättigten $C_{16}$—$C_{18}$-Fettalkohole, insbesondere als Gemisch aus gleichen Teilen der Alkohole, sorgen ebenfalls für Geschmeidigkeit und für Glätte. Sie verleihen in dispergierter Form dem Präparat den Charakter einer Lotion und dienen somit gleichzeitig zur Viskositätseinstellung.

Der nichtionogene Emulgator, vor allem Fettalkohol- oder Alkylphenolpolyglykolether, dient zur Herstellung einer stabilen Dispersion von Paraffinöl und Fettalkohol. Bevorzugt werden kolloiddisperse Gemische aus gesättigtem $C_{16}$—$C_{18}$-Fettalkohol und gesättigtem $C_{16}$—$C_{18}$-Fettalkoholpolyglykolether mit 10—25 Ethylenoxideinheiten im Verhältnis 1 : 0,15—0,5 anstelle des getrennten Einsatzes von Fettalkohol und Polyglykolether.

Desinfizierend wirkende Jod-Komplexe sind Addukte von Jod an Polyglykolether, z. B. Alkylphenol-

2

polyglykolether oder Polyethylen-Polypropylenblockcopolymere, oder an Polyvinylpyrroliden. Derartige Produkte sind beispielsweise unter der Bezeichnung Jodophor im Handel. Ein bekanntes Produkt dieser Art ist Antarox VRO-20® der Firma GAF, Deutschland. Das erfindungsgemäße Präparat enthält 0,4—0,6 Gew.-% an Jod-Komplex, entsprechend 800—1200 ppm aktives Jod.

Die Anwendung des Präparates erfolgt in üblicher Weise als Zitzentauchmittel morgens und abends nach dem Melken sowie einmal täglich bei trocken stehenden Kühen bis zum Abkalben, sowie als Hautpflegemittel für den gesamten Euterbereich, nach Bedarf. Weitere Anwendungsmöglichkeiten ergeben sich für die Euterpflege kleiner Wiederkäuer, sowie zur Gesäugepflege von Sauen. Es wird eine Keimreduktion am gesamten Euter erreicht und damit die Neuinfektionsrate gesenkt. Die Haut wird gleichzeitig optimal gepflegt, sie bleibt geschmeidig und widerstandsfähig gegenüber Umwelteinflüssen. Das Präparat eignet sich daher nicht nur zur regelmäßigen vorbeugenden Anwendung bei gesunden Milchviehbeständen, sondern insbesondere auch bei Mastitis-Problembeständen und zur Mastitis-Bestandssanierung.

Ein zusätzlicher Vorteil gegenüber herkömmlichen Melksalben und Lotionen besteht in der geringen Verkeimungsgefahr im Gebrauch infolge des Desinfektionsmittel-Gehaltes.

Beispiel

Als Zitzentauchmittel sowie zur Euterpflege von Milchkühen eignet sich ein Präparat folgender Zusammensetzung:

91,4 Gew.-% Kondenswasser
3,0 Gew.-% Glycerin DAB 7
3,0 Gew.-% Paraffinöl dickflüssig DAB 7
2,0 Gew.-% colloiddisperses Gemisch aus $C_{16}$—$C_{18}$-Fettalkohol und $C_{16}$—$C_{18}$-Fettalkohol. 20 EO-Addukt im Verhältnis 1 : 0,2
0,6 Gew.-% Jod-Komplex (Antarox VRO-20®).

Das Mittel enthält 1200 ppm aktives Jod und wird auch bei langdauernder Anwendung an den Zitzen und auf dem Euter gut vertragen.

**Patentanspruch**

Präparat zur Zitzendesinfektion und Euterpflege bei Milchvieh, insbesondere Rindern, auf der Basis von jodhaltigen Desinfektionsmitteln und hautpflegenden Substanzen, dadurch gekennzeichnet, daß das Präparat eine wäßrige Emulsion mit erhöhter Viskosität folgender Zusammensetzung ist:

84,4—91,6 Gew.-% salzfreies Wasser
3—5 Gew.-% Glycerin
3—5 Gew.-% Paraffinöl
1—2,5 Gew.-% gesättigte $C_{16}$—$C_{18}$-Fettalkohole
1—2,5 Gew.-% nichtionogener Emulgator
0,4—0,6 Gew.-% desinfizierend wirkender Jod-Komplex

wobei der Gehalt an aktivem Jod 800—1200 ppm und die Viskosität 25—35 sec/20° C im Fordbecher/ 4 mm-Düse beträgt (entspricht etwa 45—90 m Pa · s).

**Claim**

A teat-disinfecting and udder-care preparation for dairy cattle, especially cows, based on iodine-containing disinfectants and skin-care substances, characterized in that the preparation contains a high-viscosity aqueous emulsion having the following compositon:

from 84.4 to 91.6% by weight of salt-free water,
from 3 to 5% by weight of glycerine,
from 3 to 5% by weight of paraffin oil,
from 1 to 2.5% by weight of saturated $C_{16}$—$C_{18}$ fatty alcohols,
from 1 to 2.5% by weight of nonionic emulsifier,
from 0.4 to 0.6% by weight of disinfecting iodine complex,

the active iodine content amounting to between 800 and 1200 ppm and the viscosity to between 25 and 35 secs./20° C, as measured in a 4 mm orifice Ford cup (corresponding to approximately 45—90 m Pa · s).

**Revendication**

Préparation pour la désinfection des trayons et pour les soins du pis dans le bétail laitier, en particulier les bovidés, à base d'agents désinfectants contenant del'iode et de substances pour les soins de la peau, caractérisée en ce que la préparation est une émulsion aqueuse à haute viscosité de composition suivante:

84,4—91,6% en poids d'eau exempte de sels
3—5% en poids de glycérine
3—5% en poids d'huile de paraffine
1—2,5% en poids d'alcools gras saturés en $C_{16}$—$C_{18}$
1—2,5% en poids d'émulsifiant non ionogène
0,4—0,6% en poids de complexe iodé à action désinfectante

la teneur en iode actif s'élèvant à 800 à 1200 ppm et la viscosité à 25—35 secondes/20° C au bécher Ford à buselure de 4 mm (ce qui correspond à environ 45—90 m Pa · s).